# EUROPEAN PATENT APPLICATION

(11) **EP 4 559 469 A1**
(43) Date of publication of application: **28.05.2025**
(21) Application number: 23211594.9
(22) Date of filing: 22.11.2023
(51) Int. Cl.: A61K 35/74, A61K 9/00, A61K 31/726, A61K 31/727, A61K 31/728, A61P 17/00, C12N 1/20

(54) **COMPOSITIONS FOR PREVENTION OR TREATMENT OF DYSBIOSIS**

(71) Applicant: Aileens Pharma S.r.l., 20834 Nova Milanese (MB) (IT)
(72) Inventor: LONGO SORMANI, Sonia, 20834 Nova Milanese MI (IT); DI MARCO, Roberto Maria Antonio, 95123 Catania (IT); PETRONIO PETRONIO, Giulio, 95127 Catania (IT); CUTULI, Marco Alfio, 86100 Campobasso (IT); MAGNIFICO, Irene, 86170 Isernia (IT); VENDITTI, Noemi, 86021 Bojano (IT); GUARNIERI, Antonio, 86029 Trivento (IT); RUSSO, Claudio, 86010 Ferrazzano (IT); DI NARO, Maria, 95125 Catania (IT); NICOLOSI, Daria, 95125 Catania (IT)
(74) Representative: Modiano, Micaela Nadia

(57) **Abstract**

The present invention relates to a composition for preventing or treating the dysbioses caused by toxins released from bacterial extracellular vesicles, wherein the composition contains a selected strain of *Cutibacterium acnes* or a conjugate between the cell wall of the selected strain of *Cutibacterium acnes* or a portion thereof and a mucopolysaccharide, preferably hyaluronic acid or a salt thereof.

## Description

### SECTOR OF THE INVENTION

The present invention relates to a composition for the treatment of dysbioses, in particular caused by toxins released from extracellular vesicles secreted by microorganisms, in particular pathogenic bacteria.

The present invention originates in the sector of cellular and molecular biology and in the pharmaceutical field, in particular in the field of dermatology and of medical devices for external use.

In particular, the present invention relates to a composition for the treatment of dysbioses, in particular cutaneous dysbioses, wherein one of the contributing factors is constituted by the release of toxins from bacterial extracellular vesicles.

### BACKGROUND ART

Extracellular vesicles, known with the acronym EV, are vesicles with membranes of dimensions measured in nanometers, spherical, bilayered, which typically are secreted by eukaryotic cells, archaea and bacteria.

Typically, extracellular vesicles constitute a highly well-maintained and advanced intercellular communication system, through which cells can exchange information in the form of lipids, proteins or, especially, of nucleic acids.

Exosomes have also been known since the early 1980s as an EV subtype that can help eliminate molecules that a cell no longer requires to perform its activities. Subsequent studies in the 1990s showed that EVs perform an action to regulate the immune system and, subsequently, it has been observed that they are also capable of transporting proteins and RNA between cells.

In recent years, research has begun to reveal the various mechanisms with which EVs can regulate biological functions, which range from homeostasis of tissues and regulation of inflammation to the growth and metastasis of tumors. In consideration of their varied biological functions and of their capacity to transport large molecules between cells, EVs offer a unique platform for developing new classes of medicines and of products with biological effect.

EVs are present in all body fluids and are released by all types of cells in the human body, including mesenchymal cells. Typically, EVs are subdivided into i) exosomes, small vesicles that are released from inside any cell through the multivesicular endosomal route, and ii) microvesicles (MVs) which are released from cells via budding of their surface membrane. There is also a third subgroup of EVs, less well studied, known as apoptotic bodies, which are formed by blebbing of dying cells and can contain various different parts of the cell.

Typically, EVs can form through two principal mechanisms.

In particular, exosomes are formed mainly by the endocytic route through invagination of the endosomal membrane, which forms multivesicular bodies (MVBs) which can fuse with the plasma membrane in order to release exosomes into the extracellular environment.

Microvesicles (MVs), by contrast, typically derive from budding and from fission of the plasma membrane outward.

All EV subtypes share a general composition of an external lipid bilayer and of various proteins, lipids and nucleic acids transported by the vesicles.

The specific content of EVs depends to a large extent on biogenesis, on the cellular source and on the culture conditions. It has been suggested that EVs are internalized in target cells through various absorption mechanisms, including fusion of the membrane and various endocytic routes, including phagocytosis, receptor-mediated endocytosis, lipid rafts-mediated endocytosis, clathrin-mediated endocytosis, caveolin-mediated endocytosis, and the macropinocytosis.

Most bacterial populations that make up the cutaneous microbiota produce EVBs. For example, *C. acnes* and *S. aureus* but also the fungus *Malassezia furfur* have been identified as EVB producers.

In the current state of the art the need is felt to have a pharmaceutical product or preparation that substantially reduces the action of toxins released from extracellular vesicles or microvesicles.

Therefore a general aim of the present invention is to provide a bacterial cell wall conjugate of a selected strain with a mucopolysaccharide in order to block or reduce the spread of extracellular vesicles and/or of toxins released therefrom.

Another object of the invention consists in the provision of a composition containing a bacterial cell wall conjugate of a selected bacterial strain of the species *Cutibacterium acnes* with a mucopolysaccharide suitable for treating dysbioses or for restoring a physiological microbiota in a subject.

### SUMMARY OF THE INVENTION

It has surprisingly been found that a selected strain of *Cutibacterium acnes* or its cellular wall or wall portion and/or a conjugate between a) bacterial cell wall (or a portion thereof) of said strain of *Cutibacterium acnes* and b) mucopolysaccharide, blocks or substantially reduces the pathogenic action of toxins contained or released from extracellular vesicles of microorganisms such as bacteria or fungi, the proliferation of which on the skin of a subject, such as a mammal, contributes to determining cutaneous dysbioses and/or atopic dermatitis. According to a first aspect, the present invention relates to a strain of *Cutibacterium acnes* deposited under accession number DSM 28251 at the International Deposit Authority Leibniz-Institut DSMZ - Deutsche Sammlung von Mikroorganismen und Zellkulturen GmbH, or its cell wall or portion, or a conjugate between said strain or a portion of the cell wall of the strain with a mucopolysaccharide, for use in the prevention or treatment of dysbioses in a mammal, preferably a human being or an animal.

Further embodiments of the strain or conjugate for the use according to the present invention are defined in the accompanying claims 2-5.

In another aspect, the invention relates to a composition as defined in any of the accompanying claims 6-15.

The strain or conjugate or a composition that contains it for the uses described herein can be administered orally or topically. According to some embodiments, when administered orally the composition containing the conjugate is suitable for treating and/or preventing dysbioses in the gastrointestinal tract. When administered topically the composition is suitable for treating dysbioses of the skin and dermatological diseases correlated thereto.

Further uses of a composition as described herein are defined in the accompanying claims 6-15.

Advantageously the use of the selected strain or its conjugate with mucopolysaccharide described herein, or the use of a composition that contains it according to an embodiment described herein, contributes to restoring the physiological microbiota of the organism in need of treatment, reducing the pathological action of microorganisms such as, for example, pathogenic bacteria of the genus *Staphylococcus,* in particular *S*. *aureus,* and/or fungi, for example *Candida.*

In particular, the cutaneous application of a composition containing the strain or conjugate according to an embodiment described herein restores the physiological cutaneous microbiota and reduces topical infections caused by bacteria and fungi, in particular *S. aureus* and *Candida albicans.*

Advantageously the application on the skin of a topical composition that contains the strain or conjugate described herein blocks and/or limits the spread on the skin, including the epidermis and dermis, of the toxins released from exovesicles, for example EVBs preferably by *Staphylococcus aureus.*

### DETAILED DESCRIPTION OF THE INVENTION

According to some aspects of the invention, the inventors have found that:
- a strain of *Cutibacterium acnes* deposited under accession number DSM 28251 or a portion or lysate of its wall
- a conjugate of said strain with a mucopolysaccharide as described herein, when administered orally or applied on the skin of an individual, substantially reduces the pathogenic action of toxins originating from extracellular vesicles of microorganisms such as bacteria or fungi that colonize the skin, causing cutaneous dysbioses and/or atopic dermatitis.

According to a first aspect, the present invention relates to a strain or conjugate of said strain with mucopolysaccharide for the uses defined in the accompanying claim 1.

Preferably the strain of *Cutibacterium acnes* deposited under accession number DSM 28251 or a portion or lysate of its wall or a conjugate between the bacterial cell wall of said strain and a mucopolysaccharide reduces the pathogenic action of toxins, preferably toxins originating from bacterial extracellular vesicles (BEVs) for example of the genus *Staphylococcus,* preferably *Staphylococcus aureus.*

BEVs derived from *S. aureus* comprise spherical vesicular structures measuring 20-200 nm enveloped in a membrane and containing proteins, DNA, RNA, toxins and cytosolic proteins, associated with the cell wall and the membrane wall, as well as glycopolymers and exoproteins, including alpha hemolysin, leukocidins, phenol-soluble modulins, superantigens and enzymes.

Bacteria of the genus *Staphylococcus,* preferably *S. aureus,* that colonize the skin secrete BEVs which show a high immunogenicity and are correlated to dysbioses in particular of the skin.

Typically, skin dysbioses is correlated with a variation in the composition and number of commensal bacteria of the skin, in particular with an increased proliferation of the bacterial pathogen *Staphylococcus aureus.*

In particular, a prevalence of *S. aureus* has been observed on the skin of subjects with atopic dermatitis (AD), with an abundance rate of 70% compared to 39% in the control group.

Skin lesions found in atopic dermatitis are characterized by the dysfunction of the cutaneous barrier and by colonization of the epidermis by *S*. *aureus.* In particular, loss of continuity of the cutaneous barrier induced by damage to keratinocytes is one of the principal causes in the etiology of AD. Through breakage of the cutaneous barrier, the antigens and allergens associated with pathogens, for example bacteria, fungi, viruses, can penetrate into the skin and subsequently influence the composition of the microbiota and/or the immune responses of the host.

*Staphylococcus aureus* is closely correlated with AD, it colonizes the skin lesions of most patients with AD and its presence is associated with an increase in the severity of the disease.

Furthermore, it has been observed that *Staphylococcus aureus* produces hemolysins, which cause cell damage by forming heptameric pores in the cell membrane. Of these, α-hemolysin is a key toxin in the triggering of AD, and the correlation between cytotoxic effects on keratinocytes and the severity of the disease is known.

Studies in murine models also describe how α-hemolysin (Hla), present in BEVs, induces breakage of the cutaneous barrier through necrosis of keratinocytes, the destruction of tight junctions and/or the increased production of pro-inflammatory mediators, inducing cutaneous inflammation comparable to that occurring in a case of AD.

In particular, alpha-hemolysin or α-toxin is a pore-forming exotoxin (PFT) and among the exotoxins produced by *S. aureus* it is the one best characterized in EVs, in that it has a cytotoxic action toward several different types of cells, including erythrocytes, epithelial cells, endothelial cells and immune cells including neutrophils, monocytes and macrophages.

In the present invention, without wishing to be bound to any theory, it is believed that α-hemolysin and BEVs represent a new target for the treatment of AD. Hemolysin alpha is on the other hand defined as the most common of the four cytolytic toxins produced by *Staphylococcus aureus.* Typically these toxins are neutralized by the immune system of the host and they lose their activity. For soluble toxins, BEVs can protect the toxins by enveloping them with the cell membrane.

*In vitro* production of BEVs depends on several factors, including the temperature of the environment, for example of the human body. In particular it has been observed that the temperature at which BEV-producing *Staphylococci*, in particular *S*. *aureus,* are grown has a considerable influence both on the composition and on the yield, and in that the membranes of the *Staphylococci*, in particular *S*. *aureus,* cultivated at 37°C are composed mainly of straight-chain and branched-chain saturated fatty acids, while at lower temperatures, for example 30°C, *Staphylococci* modify the composition of their membrane, enriching it in unsaturated fatty acids, determining a greater fluidity of the membrane. This factor plays a key role in the process of fusion with eukaryotic cellular membranes and the release of the content of BEVs.

Without wishing to be bound to any theory, in the conjugate described herein the wall of the bacterium or portion thereof is linked to the mucopolysaccharide through the formation of a bond, for example a covalent bond. In this manner the two components of the conjugate, the wall of the selected bacteria and the mucopolysaccharide, substantially form a single product and not a simple mixture of the two components.

Advantageously, in the present description, the term "conjugate" means that the two components, the bacterial cell wall or portion thereof and the mucopolysaccharide, are mutually linked, typically with the formation of a bond for example of the covalent type.

Suitable mucopolysaccharides conjugated with the bacterial cell wall of the strain described herein are physiologically acceptable mucopolysaccharides belonging to the following groups based on central disaccharide structures:
group 1: heparin/heparan sulfate (HSGAGs);
group 2: chondroitin sulfate/dermatan sulfate (CSGAGs);
group 3: keratan sulfate, chitosan;
group 4: hyaluronic acid
or physiologically acceptable salts thereof.

According to some embodiments, the mucopolysaccharides are selected from hyaluronic acid (HA) and salts thereof, chondroitin-4-sulfate (C4SA), chondroitin-6-sulfate (C6SC), chitosan, dermatan sulfate (DS-chondroitin-sulfate B), heparin sulfate (HS), heparin (HP) and keratan sulfate (KS) and physiologically acceptable salts of the above-mentioned mucopolysaccharides.

Among the physiologically acceptable mucopolysaccharides described above, hyaluronic acid or a salt thereof are preferred. Suitable salts of hyaluronic acid include salts of sodium, potassium, calcium. In some embodiments, the molecular weight of HA is 3×10⁴ and 8×10⁶ MW (T.C. Laurent et al., Fractionation of hyaluronic acid. The polydispersity of hyaluronic acid from the bovine vitreous body, Biochim. Biophys Acta, 1960, 42, 476).

Typically, the molecular weight of the mucopolysaccharides described herein, such as hyaluronic acid, is the average molecular weight (MW) that can be determined using conventional techniques like SEC-MALLS (size exclusion chromatography with multi-angle laser light scattering), or with a method such as that described in Ueno et al., 1988, Chem Pharm Bull. 36, 4971-4975; Wyatt 1993, Anal Chim Acta 272: 1-20 40; Watt Technologies 1999 "Light scattering University Dawn Course Manual" and "Dawn Eos Manual", Wyatt Technology Corp. Santa Barbara CA (USA).

According to another aspect of the invention, a composition is provided which comprises a physiologically acceptable vehicle and a conjugate of the cell wall of a bacterium or a portion thereof conjugated with a mucopolysaccharide for use in the prevention or treatment of dysbioses in a mammal, wherein said bacterium is the selected *Cutibacterium acnes* strain deposited under accession number DSM 28251 at the International Deposit Authority Leibniz-lnstitut DSMZ - Deutsche Sammlung von Mikroorganismen und Zellkulturen GmbH.

### Definitions in the present invention:

The term "DSM 28251 strain" means the bacterial strain of the *Cutibacterium* genus, *acnes* species, initially deposited as a safe deposit on 18 December 2013 (reference identification: ULTIMO) and converted to a deposit for patent purposes under the Budapest treaty on 22 December 2019 under accession number DSM 28251 at the International Deposit Authority Leibniz-Institut DSMZ - Deutsche Sammlung von Mikroorganismen und Zellkulturen GmbH; typically, *Cutibacterium acnes* is a gram-positive bacterium. A detailed description of this strain is provided in WO2021165434, the content of which is referenced herein.

The term "bacterial cell wall conjugated with mucopolysaccharide" means the cell wall of the above-mentioned strain or a portion thereof, linked to a mucopolysaccharide advantageously with the formation of a chemical bond for example of the covalent type. Without wishing to be bound to any specific theory, it is thought that the peptidoglycan, typically murein, comprised in the bacterial cell wall interacts with the mucopolysaccharides to form a chemical bond, for example of the covalent type.

The conjugation between peptidoglycans of the bacterial wall and the mucopolysaccharide occurs under the process conditions such as, for example, described in WO2022/243558 in the name of this same Applicant. For any additional characteristics of the conjugate or of the composition containing the conjugate described herein, reference is made to the content of WO2022/243558, except where otherwise indicated.

The terms "portion" or "lysate" refer to a portion of bacterial cell wall of the strain described herein.

The term "lysate", referring to the bacterial cell wall, means fragments of the cell wall obtained via destruction using any of the cell wall destruction techniques for example as described in

The term "growth medium" (synonyms: medium, growth medium/cultivation broth/growth broth) refers to a substrate containing all the compounds (factors) required by a microorganism, especially a bacterium such as a gram positive bacterium, for cellular replication resulting in the increase of the number of single cells and in the growth of the population. The factors required by microorganisms for their growth in the medium belong mainly to the categories: a source of carbon, a source of nitrogen similar (constituted both by ammonia and by free aminoacids, the latter also known as FANs), vitamins and salts (trace minerals). Typical sources of carbon are cane sugar molasses, beet molasses, extract of barley malt and extract of wheat malt.

The term "selected bacterial strain" or "strain", as mentioned herein, means the strain deposited at the DSMZ with the accession number DSM 28251.

The term "bacterial cell wall" or "wall of the bacterial strain (of the invention)" means the cell wall, or a fragment thereof or a portion thereof or a lysate thereof, of the bacterial strain of *Cutibacterium acnes* deposited under accession number DSM 28251.

The term "vehicle" as used in the present description indicates a medium, excipient, diluent with which the association of therapeutic or active ingredients is administered.

Any vehicle and/or excipient suitable for the desired form of preparation for administration to humans is envisaged for use with the compounds described in the present invention.

For the purposes of the present invention, the term "physiologically acceptable" means edible substances that are approved by the health authorities for use in pharmaceutical, nutritional or alimentary applications.

The compositions of the present invention are suitable for pharmaceutical use in mammals, in particular in humans.

The composition of the invention can assume a wide variety of forms of preparation, according to the desired administration route.

For example, for oral administration the composition can be in solid form, for example tablet, capsule, powder, granular, i.e. prolonged-release formulations of the active ingredients. Compositions in solid form, in particular in tablet form, are preferred over other types of preparations.

Preparations in solid form can comprise one or more vehicles such as for example amides, sugars, micro-crystalline cellulose, and optionally diluents, granulation agents, lubricants, binding agents, disintegration agents.

The tablets, pills, capsules, and granulates can also contain a binding agent such as tragacanth, acacia, corn starch or gelatin; excipients such as dicalcium phosphate; a disintegration agent such as corn starch, potato starch, alginic acid; a lubricant such as magnesium stearate; a sweetening agent such as sucrose, lactose or saccharine. If desired, the tablets can be coated using conventional techniques.

When the unitary pharmaceutical form is a capsule, it can contain, in addition to the materials of the type mentioned above, a liquid vehicle such as a fatty oil.

For preparations in liquid form for oral administration, such as for example for suspensions, emulsions, and solutions, a suitable vehicle can be selected from water, glycols, oils, alcohols and mixtures thereof.

In some embodiments, the composition of the invention is in liquid form, ready to be taken.

In the composition there can also be flavoring agents, preservatives, coloring agents and the like.

In some embodiments, the vegetable extracts or active ingredients contained in the composition of the present invention can be combined or mixed as active ingredients in an intimate mixture with a suitable edible vehicle and/or an excipient according to conventional pharmaceutical techniques and conventional food/nutrition industry.

The compositions for pharmaceutical or nutritional use can be suitably presented in a single pharmaceutical form and prepared using any of the methods that are well known in pharmaceutical or food technology.

In some embodiments, the compositions or preparations of the invention can contain at least 0.1% of each bioactive ingredient/substance. The percentage of active ingredient in these compositions can obviously vary, for example from approximately 1% to approximately 60% of the weight of the unit. The quantity of active compound in such compositions is such as to obtain a dose that is prophylactically or therapeutically effective.

In some embodiments, the composition of the invention further comprises one or more additional components such as additives, fillers, stabilizers, emulsifiers, texturizing agents, film-forming agents, plasticizers, wetting agents and thickeners.

Various other materials can be present as coatings or to modify the physical shape of the pharmaceutical unit. For example, tablets can be coated with shellac, sugar or both. To prevent disintegration during transit through the upper part of the gastrointestinal tract, the composition can be a formulation with an enteric coating.

A syrup or elixir can contain, in addition to the active ingredient, sucrose as a sweetening agent, methyl and propyl parabens as preservatives, a coloring agent, and a flavoring agent such as cherry or orange flavor.

In some embodiments, in the compositions of the present invention, the active ingredients are normally formulated in dosage units. A dosage unit can contain from 0.1 to 1,000 mg of active ingredient or of vegetable extract containing the active ingredient per dosage unit for daily administration.

In some embodiments, the dosage unit of the composition of the invention is a sachet that can contain from 1 to 50 g, from 5 to 30g of composition.

In some embodiments, the formulation will contain amounts of active ingredients that will depend on the severity of the dysbioses, on the associated symptoms, on the condition, on the further treatments being administered, on the individual state of health, and on the response to the combination of active ingredients. In some embodiments the dose is in the range of from 0.001% by weight to approximately 60% by weight of the formulation.

According to some embodiments the composition of the invention is a food supplement.

The present invention is further illustrated in detail by the following example.

### EXAMPLE 1

### Rationale of the study

Starting from this assumption, in order to better explain the mechanism of action of the conjugate according to certain aspects of the invention, also referred to hereinafter as LimpiAD, we believe it is necessary to investigate the role that this medical device can have in blocking and/or limiting the emission of BEVs. This is a very complex study and one that requires both a high level of specialization and lengthy times for implementation.

Therefore the experimental plan will be divided into three separate study phases.

### STAGE 1

### Selection and growth of the strains

At least three strains of *S*. *aureus,* fully characterized in the literature, will be selected as producers of BEVs and of α-hemolysin [4]. From an initial analysis of the literature, several strains available from databanks are potentially useful for our purpose. Before beginning the experimental set-up it will be necessary to acquire the strains and test their effective toxin production inside BEVs.

### Potential candidates: S. aureus NCTC 8325, S. aureus ATCC 14458, S. aureus ATCC 6538P, S. aureus DSM 20491

### Isolation of EVs

The methods of extraction and purification of BEVs are known to us and validated by the literature. That notwithstanding, to our knowledge, no fully-validated test has been devised for evaluating the possible interactions between a formula such as that of LimpiAD and BEVs, and furthermore for the full performance of the test it will be necessary to show that the selected isolation method does not alter the biological characteristics of the content of the BEVs. In light of these opening remarks, it will therefore be necessary to perform a series of preliminary trials in order to optimize the most suitable experimental conditions for the definitive tests.

### Glucose gradient ultracentrifugation

Bacterial cells will be eliminated through low-speed centrifugation. The supernatant of the culture contains vesicles, secreted soluble proteins, and proteins released by the cell lysis. In order to ensure the removal of all the cells, the supernatant will be sterile-filtered (0.45 µm). The vesicles in the cell-free supernatants will be pelletized through high-speed centrifugation (≥40,000 × g). The secreted proteins are co-pelletized with the BEVs if they are associated with the vesicles or if they are of large dimensions or are abundant. In light of this, in order to purify the vesicles of the secreted proteins, the pellets will be suspended once again in a buffer and loaded on the bottom of a density gradient. The vesicles will float further upward than the soluble proteins [9].

### Poly-L-lysine S

This isolation strategy is based on a cationic polymer, poly-L-lysine (PL) [Poly-L-lysine hydrobromide; mol. wt 70;00 Sigma S-P1274], in order to precipitate the BEVs at a relatively low centrifugation speed (10,000 × g). With respect to the standard ultracentrifugation strategy, the precipitated BEVs can be recovered by adjusting the pH and the ionic strength of the culture medium, followed by an ultrafiltration step to remove ε-PL and obtain the buffer exchange [10].

### Characterization of BEVs and of the toxins of interest

### Tracking analysis

The particle size distribution of the isolated BEVs will be determined using nanoparticle tracking analysis (NTA). Nanoparticle tracking analysis is a method of viewing and analyzing particles in liquids. NTA suspends small particles (-10-1000 nm) in a liquid, making it possible to determine a size distribution profile. All the analyses will use the same set of parameters in order to ensure comparable results [10].

### Total proteinic concentration

The proteinic concentration of the BEVs will be quantified using the BCA protein dosing kit.

### Selection of fluorescent dyes

In order to obtain a correct coloring of the extracted BEVs, dyes will be evaluated which are weakly fluorescent in water, but are highly fluorescent and sufficiently photostable if incorporated in membranes. To this end, a series of long-chain lipophilic carbocyanine dyes, belonging to the Dil family (1,1'-Dioctadecyl-3,3,3',3'-Tetramethylindocarbocyanine Perchlorate), will be tested. Some prominent members of the series comprise: Dil, also known as DiIC18(3); DiO, also known as DiOC18(3); DiD, also known as DiIC18(5); and DiR, also known as DiIC18(7), which respectively have orange, green, red and infrared fluorescence [11].

### Biological testing of the content

### Western Blot and ELISA assay for the presence of alpha hemolysin

To confirm the presence of the toxin, be it in soluble/free form or inside the BEVs, special Western Blot and ELISA protocols will be provided that entail the use of specific anti α-toxin antibodies.

### Validation tests in vitro, ex vivo and in vivo

### In vitro: cell cultures and cytotoxicity assays

The immortal keratinocyte cell line (HaCaT) which presents characteristics similar to the primary cultures of keratinocytes and is already known from studies of interactions between *S. aureus* and keratinocytes, will be used for the *in vitro* validation.

The vitality of keratinocytes infected with BEVs alone or pre-treated with LimpiAD will be evaluated both with the thiazolyl blue tetrazolium bromide (MTT) assay, and through evaluation of the activity of the lactate dehydrogenase (LDH) enzyme.

### Ex vivo: biopsies of pig skin

The *ex vivo* model will be optimized on pig skin (*Sus scrofa domesticus*), already used for previous studies on the activity of *S*. *aureus* catabolites, which by virtue of its exceptional histological and physiological similarity to human skin, makes it an ideal candidate for cutaneous studies [7].

### STAGE 2

### Fine and ultrastructural in vivo characterization of BEVs (before and after contact with LimpiAD)

Given the importance of the composition of the membranes of BEVs in the binding with the cellular target and therefore the release of the cargo (alpha hemolysin), before the biological assays an evaluation will be made of the interfering capacity of LimpiAD in the production and in the proteinic composition of the BEVs and/or of their content.

### Analysis of the proteinic composition

The isolated BEVs will be separated using SDS-PAGE (10%). The bands on gel will be excised, digested and analyzed using LC-MS/MS. The MS/MS spectra of each LC-MS/MS run will be compared with data sets present in public databases [10].

### Evaluation of microscopy methods

Owing to their small dimensions, direct observation of BEVs requires suitable microscopy methods. To this end, various different strategies will be evaluated: transmission electron microscopy (TEM), scanning electron microscopy (SEM), or even atomic force microscopy [12].

### STAGE 3

### Final in vivo validation tests

### Murine models

The results obtained from *in vitro* and *ex vivo* models will be validated by *in vivo* assays with murine models. Suitable murine models of epicutaneous type will be employed to evaluate the role of toxins of *S. aureus* released by exovesicles in the pathogenesis of AD.

## Claims

1. A strain of *Cutibacterium acnes* deposited under accession number DSM 28251 at the International Deposit Authority Leibniz-lnstitut DSMZ - Deutsche Sammlung von Mikroorganismen und Zellkulturen GmbH, or its cell wall or portion or conjugate between the cell wall of said strain or a portion thereof and a mucopolysaccharide, for use in the prevention or treatment of dysbioses in a mammal, preferably a human being or an animal.

2. The strain or conjugate for the use according to claim 1, wherein the mucopolysaccharide is selected from the group consisting of heparin, heparan sulfate, chondroitin sulfate, dermatan sulfate, keratan sulfate, chitosan, hyaluronic acid and/or physiologically acceptable salts thereof.

3. The strain or conjugate for the use according to claim 1 or 2, wherein the mucopolysaccharide is selected from hyaluronic acid (HA), chondroitin-4-sulfate (C4SA), chondroitin-6-sulfate (C6SC), chitosan, dermatan sulfate (DS-chondroitin-sulfate B), heparin sulfate (HS), heparin (HP) and keratan sulfate (KS) and physiologically acceptable salts thereof.

4. The strain or conjugate for the use according to any one of claims 1 to 3, wherein said mucopolysaccharide is hyaluronic acid or a physiologically acceptable salt thereof.

5. The strain or conjugate for the use according to any one of claims 1 to 4, wherein the dysbioses is caused by toxins released from exovesicles secreted by microorganisms, preferably bacterial exovesicles.

6. A composition comprising a strain or conjugate for the use according to any one of claims 1 to 5, and a physiologically acceptable vehicle.

7. The composition for the use according to claim 6, which comprises oral administration or topical administration.

8. The composition for the use according to claim 6 or 7 for use in the treatment of dysbioses of the gastrointestinal tract or of skin dysbioses.

9. The composition for the use according to any one of claims 7, 8, wherein the dysbioses is caused by toxins released from exovesicles secreted by microorganisms, preferably bacteria.

10. The composition for the use according to claim 9, wherein said bacteria belong to the genus *Staphylococcus,* preferably to the species *Staphylococcus aureus.*

11. The composition for the use according to any of claims 6-10, for use by means of topical application.

12. The composition for the use according to claim 11, for the topical application on the epidermis and/or mucous membranes of a human being or animal.

13. The composition for topical use according to any one of claims 11-12, wherein the composition is a cream, an unguent, a paste, a foam, a gel, a powder solution, a vaginal suppository or an emulsion.

14. A composition comprising a strain or cell wall of *Cutibacterium acnes* deposited under accession number DSM 28251 at the International Deposit Authority Leibniz-lnstitut DSMZ - Deutsche Sammlung von Mikroorganismen und Zellkulturen GmbH or a portion of said wall or a conjugate of said wall or portion of wall with a mucopolysaccharide for the use according to any one of claims 1 to 5 and a physiologically acceptable vehicle for use in restoring the physiological microbiota of the gastrointestinal tract and/or of the skin.

15. The composition for the use according to claim 14 in restoring the physiological microbiota of the skin by means of topical application on the skin and/or mucous membrane of a subject.
